# EUROPÄISCHE PATENTANMELDUNG

(11) **EP 3 536 955 A1**
(43) Veröffentlichungstag der Anmeldung: **11.09.2019**
(21) Anmeldenummer: 18160699.7
(22) Anmeldetag: 08.03.2018
(51) Int. Cl.: F04B 43/00, F04B 49/20

(54) **ANTRIEBSVORRICHTUNG FÜR EINE MEMBRANFLUIDPUMPE UND BETRIEBSVERFAHREN**

(71) Anmelder: Berlin Heart GmbH, 12247 Berlin (DE)
(72) Erfinder: STEINGRÄBER, Robert, 14055 Berlin (DE)
(74) Vertreter: Pfenning, Meinig & Partner mbB

(57) **Zusammenfassung**

Die Erfindung betrifft eine Antriebsvorrichtung für eine Membranfluidpumpe, umfassend eine Arbeitspumpe, wobei die Arbeitspumpe zum Antreiben der Membranfluidpumpe über eine Druckleitung mit der Membranfluidpumpe verbunden ist, und wobei die Arbeitspumpe einen Hohlzylinder und einen darin axial zwischen zwei Umkehrpunkten oszillierbaren Arbeitskolben zum Verkleinern und Vergrößern eines mit der Druckleitung in einer Druckaustauschverbindung stehenden Arbeitsraums in der Arbeitspumpe aufweist, und eine Steuereinheit zum Steuern einer Bewegung des Arbeitskolbens zwischen den Umkehrpunkten, dadurch gekennzeichnet, dass die gesteuerte Bewegung des Arbeitskolbens drei zeitlich aufeinanderfolgende Phasen umfasst, wobei in einer ersten Phase der Arbeitskolben auf eine Geschwindigkeit beschleunigt wird, die größer ist als eine Geschwindigkeit am Ende der ersten Phase, in einer zweiten Phase der Arbeitskolben derart bewegt wird, dass eine vorbestimmte Geschwindigkeit des Arbeitskolbens, ein vorbestimmter Relativdruck im Arbeitsraum oder eine vorbestimmte Kraft des Arbeitskolbens weitgehend konstant gehalten wird, und in einer dritten Phase der Arbeitskolben mit einer negativen Beschleunigung bewegt wird.

## Beschreibung

Die vorliegende Erfindung betrifft eine Antriebsvorrichtung für eine Membranfluidpumpe, ein Pumpensystem und ein Herzunterstützungssystem jeweils enthaltend eine derartige Antriebsvorrichtung sowie ein Verfahren zum Betrieb einer derartigen Antriebsvorrichtung.

Im Stand der Technik sind Antriebsvorrichtungen für Membranfluidpumpen bekannt, die als Antriebsvorrichtungen für Membranblutpumpen in Herzunterstützungssystemen verwendet werden. Diese Antriebsvorrichtungen weisen einen Pneumatik-Zylinder auf, der über eine Druckleitung mit der Membranblutpumpe verbunden ist. In dem Pneumatik-Zylinder kann ein Kolben oszillatorisch zwischen zwei Umkehrpunkten hin und her bewegt werden. Durch die Bewegung des Kolbens verringert oder vergrößert sich das Volumen eines mit der Druckleitung in einer Druckaustauschverbindung stehenden Arbeitsraums innerhalb des Pneumatik-Zylinders. Der Arbeitsraum weist weiterhin ein Ausgleichsventil auf, über das der Arbeitsraum zum Luftaustausch mit der Umgebung verbunden ist. Mit diesem Ventil kann bei negativem Relativdruck im Arbeitsraum Luft angesaugt werden und bei positivem Relativdruck Luft abgelassen werden. Der Kolben wird durch eine Spindel bewegt, die mit einem elektrischen Motor angetrieben wird.

Insbesondere ist eine Antriebsvorrichtung bekannt, die für große Blutpumpen ab 60 ml Fördervolumen zum Einsatz kommt.

Die Aufgabe der vorliegenden Erfindung ist es daher, eine neue Antriebsvorrichtung für Membranfluidpumpen bereitzustellen, die sich insbesondere auch für kleine Membranfluidpumpen eignet.

Weiterhin sollen ein entsprechendes Verfahren zum Betrieb der Antriebsvorrichtung, ein entsprechendes Pumpensystem und ein entsprechendes Herzunterstützungssystem enthaltend die Antriebsvorrichtung bereitgestellt werden.

Die Aufgabe wird durch eine Antriebsvorrichtung nach Anspruch 1, ein Pumpensystem nach Anspruch 13, ein Herzunterstützungssystem nach Anspruch 14 sowie ein Verfahren zum Betrieb der Antriebsvorrichtung nach Anspruch 15 gelöst. Vorteilhafte Weiterbildungen der erfindungsgemäßen Antriebsvorrichtung und des erfindungsgemäßen Verfahrens sind in den abhängigen Ansprüchen 2 bis 12 bzw. 16 bis 24 aufgeführt.

Die erfindungsgemäße Antriebsvorrichtung für eine Membranfluidpumpe, umfasst eine Arbeitspumpe, wobei die Arbeitspumpe zum Antreiben der Membranfluidpumpe über eine Druckleitung mit der Membranfluidpumpe verbunden ist, und wobei die Arbeitspumpe einen Hohlzylinder und einen darin axial zwischen zwei Umkehrpunkten oszillierbaren Arbeitskolben zum Verkleinern und Vergrößern eines mit der Druckleitung in einer Druckaustauschverbindung stehenden Arbeitsraums in der Arbeitspumpe aufweist.

Weiterhin umfasst die erfindungsgemäße Arbeitspumpe eine Steuereinheit zum Steuern einer Bewegung des Arbeitskolbens zwischen den Umkehrpunkten. Unter Steuern einer Bewegung des Arbeitskolbens wird hier eine gezielte Beeinflussung der Bewegung des Arbeitskolbens verstanden, sodass eine vorgegebene Bewegung des Arbeitskolbens bzw. ein vorgegebener Zustand der Arbeitspumpe erreicht wird. Insbesondere wird unter Steuern der Bewegung des Arbeitskolbens eine Regelung zum Erreichen der vorgegebenen Bewegung bzw. des vorgegebenen Zustands verstanden.

Die erfindungsgemäße Antriebsvorrichtung zeichnet sich dadurch aus, dass die gesteuerte Bewegung des Arbeitskolbens drei zeitlich aufeinanderfolgende Phasen umfasst, wobei in einer ersten Phase der Arbeitskolben auf eine Geschwindigkeit beschleunigt wird, die größer ist als eine Geschwindigkeit am Ende der ersten Phase, in einer zweiten Phase der Arbeitskolben derart bewegt wird, dass eine vorbestimmte Geschwindigkeit des Arbeitskolbens, ein vorbestimmter Relativdruck im Arbeitsraum oder eine vorbestimmte Kraft des Arbeitskolbens weitgehend konstant gehalten wird, und in einer dritten Phase der Arbeitskolben mit einer negativen Beschleunigung bewegt wird.

Unter Beschleunigen wird im Folgenden eine positive Beschleunigung, also eine Zunahme der Geschwindigkeit in die Bewegungsrichtung verstanden, falls das Vorzeichen der Beschleunigung nicht explizit angeben ist. Als Relativdruck wird hier der zum Umgebungsdruck relative Druck im Arbeitsraum bezeichnet. Die Kraft des Arbeitskolbens kann insbesondere eine Last und eine Trägheit des Arbeitskolbens einschließen.

Die erste bis dritte Phase, auch als Kompressionsphase, Pumpphase und Bremsphase bezeichnet, werden dabei innerhalb eines halben Pumpzyklus durchlaufen, also einmal in einer Entleerungsphase und einmal in der Füllungsphase. Unter der Entleerungsphase wird hier die Phase verstanden, in der die Membranfluidpumpe durch die Bewegung des Arbeitskolbens entleert wird. Unter der Füllungsphase wird hier die Phase verstanden, in der die Membranfluidpumpe durch die Bewegung des Arbeitskolbens gefüllt wird. Die erste, zweite und dritte Phase können dabei unterschiedliche Zeitdauern aufweisen. Insbesondere kann die erste Phase eine Zeitdauer von ≥ 30% und/oder ≤ 45%, vorzugsweise ≥ 35% und/oder ≤ 40% der Zeitdauer einer Pumpzyklushälfte aufweisen, die zweite Phase eine Zeitdauer von ≥ 35% und/oder ≤ 50%, vorzugsweise ≥ 40% und/oder ≤ 45% der Zeitdauer einer Pumpzyklushälfte aufweisen und die dritte Phase eine Zeitdauer von ≥ 1% und/oder ≤ 16%, vorzugsweise ≥ 5% und/oder ≤ 10%, weiter bevorzugt 8% der Zeitdauer einer Pumpzyklushälfte aufweisen. Die Zeitdauern der Kompressions- und der Pumpphase können auch variabel sein und/oder von Zustandsgrößen der Arbeitspumpe abhängen. Beispielsweise können die Zeitdauern vom Relativdruck im Arbeitsraum oder von einem abgefahrenen Kolbenhub abhängen. Beispielsweise kann in einem Fall, in dem der Arbeitskolben in der zweiten Phase, also der Pumpphase, derart bewegt wird, dass ein vorbestimmter Relativdruck konstant gehalten wird, die vorhergehende Kompressionsphase so lange andauern bis eine vorbestimmte Kolbenposition im Arbeitsraum erreicht ist.

Weiterhin können die drei Phasen jeweils durch eine Übergangsphase voneinander getrennt sein, wobei die erste und die zweite Phase durch eine erste Übergangsphase, die zweite und die dritte Phase durch eine zweite Übergangsphase und die dritte und die erste Phase durch eine dritte Übergangsphase voneinander getrennt sind. Die Übergangsphasen dienen der Stetigkeit der Beschleunigung des Arbeitskolbens, so dass eine ruckfreie bzw. belastungsarme Bewegung des Arbeitskolbens ermöglicht wird. Die Übergangsphasen können gleiche oder verschiedene Zeitdauern aufweisen. Im Falle gleicher Zeitdauern der Übergangsphasen kann die Zeitdauer einer Übergangsphase ≥ 2% und/oder ≤ 10%, insbesondere ≥ 3% und/oder ≤ 6%, besonders bevorzugt 4% der Zeitdauer einer Pumpzyklushälfte betragen.

Ferner ist es denkbar, dass zwei oder alle der ersten bis dritten Phase direkt aufeinanderfolgen. Die Summe der Zeitdauern aller auftretenden Phasen entspricht dann der Zeitdauer der Entleerungs- bzw. Füllungsphase. Die Zeitdauer der Entleerungs- bzw. Füllungsphase wird auch als Entleerungs- bzw. Füllungsdauer bezeichnet.

Die erfindungsgemäße Antriebsvorrichtung eignet sich insbesondere zum Antreiben von kleinen Membranfluidpumpen. Das Steuern der Bewegung des Arbeitskolbens der Antriebsvorrichtung gemäß den drei Phasen ermöglicht eine hohe Pumpleistung und eine geringe der Gefahr der Blutschädigung durch die Membranfluidpumpe. Die hohe Pumpleistung wird insbesondere dadurch erreicht, dass zunächst in der ersten Phase zügig eine bestimmte Pumpgeschwindigkeit, ein bestimmter Pumpdruck oder eine bestimmte Pumpkraft erreicht wird. Hierbei muss anfangs nur wenig Energie durch die Antriebsvorrichtung aufgebracht werden, da der Relativdruck im Arbeitsraum zu Beginn der ersten Phase noch gering ist und die Bewegung des Arbeitskolbens unterstützt. Dies erhöht den Wirkungsgrad der Antriebsvorrichtung. Die zweite Phase konstanter Pumpgeschwindigkeit, konstanten Pumpdrucks oder konstanter Pumpkraft ermöglicht eine effiziente Übertragung der Pumpenergie auf die Membranfluidpumpe, ein gleichmäßiges Pumpen der Membranfluidpumpe und somit einen gleichmäßigen Fluss des Fluids aus der und in die Membranfluidpumpe. Durch den gleichmäßigen Fluss des Fluids wird insbesondere auch die Gefahr der Blutschädigung verringert. In der dritten Phase wird der Arbeitskolben abgebremst, um daraufhin nach dem Umkehren in die entgegengesetzte Richtung bewegt zu werden.

Die Steuereinheit kann insbesondere eingerichtet sein, die Bewegung des Arbeitskolbens so zu steuern, dass in der ersten Phase der Arbeitskolben auf eine Geschwindigkeit beschleunigt wird, die mindestens 1,2 mal, vorzugsweise mindestens 1,6 mal und höchstens 15 mal, vorzugsweise höchstens 7 mal größer ist als die Geschwindigkeit am Ende der ersten Phase. Weiterhin kann die Steuereinheit eingerichtet sein, die Bewegung des Arbeitskolbens so zu steuern, dass der Arbeitskolben in der ersten Phase mit einer linear abnehmenden Beschleunigung bewegt wird. Hierdurch wird ein positions- oder geschwindigkeitsgeregelter Betrieb möglich, in welchem Unterschiede durch die Fertigung oder durch Verschleiß kompensiert werden können. Außerdem kann eine Spitzengeschwindigkeit (Spitzendrehzahl) auf einen Zielwert begrenzt werden und die Positionsvorgabe mit nur einem Parameter an die angeschlossene Pumpen-Kanülen-Kombination angepasst werden.

Die Steuereinheit kann weiterhin eingerichtet sein, die Bewegung des Arbeitskolbens so zu steuern, dass der Arbeitskolben in der dritten Phase mit einer weitgehend konstanten, negativen Beschleunigung bewegt wird. Dies ermöglicht es, den Arbeitskolben langsam abzubremsen, wodurch eine Spitzenbeschleunigung und ein Strom begrenzt werden können.

Die dritte Phase kann insbesondere mit einem Stillstand des Arbeitskolbens enden, nach welchem der Arbeitskolben in die entgegengesetzte Richtung weiterbewegt wird. Stillstand bedeutet hier eine Geschwindigkeit von Null, jedoch nicht zwingend eine Beschleunigung gleich Null.

Vorzugsweise kann die Antriebsvorrichtung einen elektrischen Motor zum Antreiben des Arbeitskolbens umfassen. Insbesondere kann der elektrische Motor eine Spindel, insbesondere eine Kugelgewindespindel oder einen Kugelgewindetrieb, umfassen, die eingerichtet ist, ein Drehmoment des Motors auf den Arbeitskolben so zu übertragen, dass der Arbeitskolben eine translatorische Bewegung zwischen den Umkehrpunkten ausführt. Zwischen einer Drehzahl n der Spindel und der Geschwindigkeit v des Arbeitskolbens besteht vorzugsweise eine feste kinematische Beziehung der Form v = const. * n.

Die Steuereinheit kann eingerichtet sein, die Bewegung des Arbeitskolbens gemäß einer zeitlichen Positionsvorgabe für den Arbeitskolben zu steuern, wobei in der zweiten Phase die Geschwindigkeit des Arbeitskolbens weitgehend konstant gehalten wird. Unter der Steuerung der Bewegung des Arbeitskolbens gemäß der zeitlichen Positionsvorgabe wird insbesondere eine Regelung (auch mit Positionsregelung bezeichnet) verstanden, in der die zeitliche Positionsvorgabe eine Regelgröße ist. Eine Steuerung der Bewegung gemäß einer Positionsvorgabe weist eine geringe Störanfälligkeit gegenüber Laständerungen und Leckagen auf. Beispielsweise führt ein fehlerhafter Relativdruck im Arbeitsraum nicht unmittelbar zu einer fehlerhaften Bewegung des Arbeitskolbens. Ein weiterer Vorteil der Steuerung der Bewegung des Arbeitskolbens gemäß einer Positionsvorgabe besteht darin, dass der Arbeitskolben auch bei steigender Last unabhängig vom Druck gemäß der Positionsvorgabe gesteuert wird. Bei der Steuerung gemäß einer Druckvorgabe würde das geförderte Luftvolumen in diesem Fall verringert werden. Bei der Steuerung gemäß Positionsvorgabe geht der Blutfluss bei steigender Last somit nicht so stark zurück, wie dies bei der Steuerung gemäß Druckvorgabe der Fall wäre. Ferner können mechanische Unterschiede verschiedener Arbeitspumpen kompensiert werden, wodurch der Blutfluss reproduzierbarer und gleichmäßiger wird.

Ergänzend oder alternativ kann die Steuereinheit eingerichtet sein, die Bewegung des Arbeitskolbens gemäß einer zeitlichen Relativdruckvorgabe für den Arbeitsraum zu steuern, wobei in der zweiten Phase der Relativdruck im Arbeitsraum weitgehend konstant gehalten wird. Unter der Steuerung der Bewegung des Arbeitskolbens gemäß der zeitlichen Relativdruckvorgabe wird insbesondere eine Regelung (auch mit Druckregelung oder Relativdruckregelung bezeichnet) verstanden, in der die zeitliche Relativdruckvorgabe eine Regelgröße ist. Eine Steuerung gemäß einer Druckvorgabe ist insofern vorteilhaft, da Benutzern von Antrieben mit Drucktanks diese Art der Steuerung bereits aus dem Stand der Technik bekannt ist und sich Parameter intuitiver einstellen lassen. Ein zusätzlicher Vorteil der Druckregelung besteht darin, dass es möglich ist, in der Füllungsphase einen gleichmäßig niedrigen Füllungsdruck einzustellen und dadurch die Gefahr der Blutschädigung zu verringern. Weiterhin kann die Zeitdauer der Pumpphase variabel gehalten werden, sodass der Beginn und/oder das Ende der Pumpphase von einer Position des Arbeitskolbens abhängig gemacht werden kann.

Die Steuereinheit kann ergänzend oder alternativ zu einer Steuerung gemäß der zeitlichen Positions- und/oder Relativdruckvorgabe eingerichtet sein, die Bewegung des Arbeitskolbens gemäß einer zeitlichen Kraftvorgabe für den Arbeitskolben zu steuern, wobei in der zweiten Phase die Kraft für den Arbeitskolben weitgehend konstant gehalten wird. Unter der Steuerung der Bewegung des Arbeitskolbens gemäß der zeitlichen Kraftvorgabe wird insbesondere eine Regelung (auch mit Kraftregelung bezeichnet) verstanden, in der die zeitliche Kraftvorgabe eine Regelgröße ist. Wie bei der Steuerung gemäß der Relativdruckvorgabe lassen sich Parameter intuitiver einstellen. Wie bei der Steuerung gemäß der Positionsvorgabe kommt der Fluidfluss auch bei der Steuerung gemäß einer Kraftvorgabe durch Laständerungen nicht sofort zum Erliegen, da die für die Beschleunigung des Arbeitskolbens nicht erforderliche Kraft für eine Erhöhung des Relativdrucks am Anfang einer Pumpzyklushälfte genutzt wird. Ferner können mechanische Unterschiede verschiedener Arbeitspumpen kompensiert werden, wodurch der Blutfluss reproduzierbarer und gleichmäßiger wird.

Die Steuereinheit kann ferner eingerichtet sein, die Steuerung gemäß der Positionsvorgabe, die Steuerung gemäß der Relativdruckvorgabe und/oder gemäß der Kraftvorgabe zu kombinieren. Beispielsweise kann die Steuereinheit eingerichtet sein, die zeitliche Positionsvorgabe basierend auf der zeitlichen Relativdruckvorgabe zu bestimmen und die Bewegung des Arbeitskolbens gemäß der Positionsvorgabe zu steuern. Durch eine derartige unterlegte Positionsvorgabe ist es möglich, mechanische Unterschiede auszugleichen, ohne Druckfeedback einen Mindestdruck aufzubauen, Randbedingungen für die Positionsvorgabe und die Geschwindigkeitsvorgabe zu berücksichtigen, ein Schlagvolumen, also das in einer Pumpzyklushälfte von dem Arbeitskolben überstrichene Volumen des Arbeitsraums, und ein Totvolumen, also ein nicht vom Arbeitskolben überstrichenes Volumen des Arbeitsraumes zwischen einem Umkehrpunkt am Ende der Entleerungsphase und der Zylinderwand, einen Zieldruck (Maximaldruck) für die Pumpphase, eine Strom- und Drehzahlreserve, einen schnellen Druckwechsel, eine Mindestdrehzahl und eine ruckfreie Bewegung vorzugeben.

Weiterhin kann die Steuereinheit eingerichtet sein, zwischen der Steuerung der Bewegung des Arbeitskolbens gemäß der zeitlichen Positionsvorgabe, der Steuerung der Bewegung des Arbeitskolbens gemäß der zeitlichen Relativdruckvorgabe und/oder der Steuerung der Bewegung des Arbeitskolbens gemäß der zeitlichen Kraftvorgabe umzuschalten. In diesem Fall kann die Steuereinheit eingerichtet sein, die Bewegung des Arbeitskolbens in der ersten und dritten Phase gemäß der Positionsvorgabe und in der zweiten Phase gemäß der Relativdruckvorgabe und/oder gemäß der Kraftvorgabe zu steuern. Dies ermöglicht die Vorteile der verschiedenen Regelungskonzepte Positions-, Relativdruck- und Kraftregelung in jeder der drei Phasen zu nutzen. Dadurch kann die Arbeitspumpe die vorgegebene Positions-, Relativdruck- und Kraftvorgabe in hohem Maße reproduzierbar und stabil durchlaufen und kann eine Spitzendrehzahl und/oder eine Spitzenbeschleunigung einhalten, was mit einer Druck- oder Kraftregelung bei niedrigen Relativdrücken schwer erzielbar ist. Durch die Relativdruck- oder Kraftregelung in der zweiten Phase orientiert sich die Arbeitspumpe in der zweiten Phase direkt am Anwenderziel.

Gemäß einer weiteren Variante kann die Steuereinheit eingerichtet sein, die Positionsvorgabe basierend auf einer Verlaufsmessung des Relativdrucks aus vorhergehenden Pumpzyklen zu bestimmen. Der Vorteil dieser iterativ lernenden Positionsregelung ist, dass kein Umschalten zwischen verschiedenen Reglern für die Kolbenbewegung erforderlich ist, sondern durchgängig die robust stabile Positionsregelung zum Einsatz kommt. Durch die Nutzung der gemessenen Druckverläufe wird jedoch die Positionsvorgabe langsam so angepasst, dass die Druckziele des Anwenders trotz langsamer Laständerungen erreicht werden. Kurzzeitigen Störungen wird im Gegensatz zur Druckregelung nicht gefolgt.

Ferner kann die Steuereinheit eingerichtet sein, die zeitliche Kraftvorgabe basierend auf der zeitlichen Positionsvorgabe zu bestimmen und die Bewegung des Arbeitskolbens gemäß der Kraftvorgabe zu steuern. Beispielsweise kann mittels eines Positionssensors eine Differenz zwischen einem vorgegebenen Kolbenhub und einem erreichten Kolbenhub gemessen werden und die Kraft des Arbeitskolbens für den nächsten Pumpzyklus so angepasst werden, dass die Differenz im nächsten Pumpzyklus verringert wird.

Weiterhin kann die Steuereinheit eingerichtet sein, die Bewegung des Arbeitskolbens gemäß einer zeitlichen Relativdruckvorgabe für den Arbeitsraum zu steuern und einer zeitlichen Geschwindigkeitsvorgabe für den Arbeitskolben zu steuern. Hierdurch kann auf einfache Weise eine Maximaldrehzahl der Spindel festgelegt werden und so eine sichere Vorgabe für die Maximaldrehzahl eingehalten werden.

Die zeitliche Positionsvorgabe kann zusätzlich eine zeitliche Geschwindigkeitsvorgabe und/oder eine zeitliche Beschleunigungsvorgabe beinhalten. Die Steuereinheit kann eingerichtet sein, die Bewegung des Arbeitskolbens gemäß der zeitlichen Positionsvorgabe, gemäß der zeitlichen Geschwindigkeitsvorgabe und/oder gemäß der zeitlichen Beschleunigungsvorgabe gleichzeitig zu steuern.

Besonders vorteilhaft ist es, wenn die Arbeitspumpe ein Ausgleichsventil zum Ändern einer Luftmasse und/oder eines Relativdrucks im Arbeitsraum umfasst. Die Steuereinheit kann dann eingerichtet sein, das Ausgleichsventil gemäß einer Vorgabe für die Luftmasse zu steuern, wobei die Vorgabe für die Luftmasse insbesondere ein Konstanthalten der Luftmasse umfassen kann. Die Steuereinheit kann auch eingerichtet sein, das Ausgleichsventil gemäß einer Vorgabe für einen mittleren Relativdruck zu steuern, wobei die Vorgabe insbesondere ein Konstanthalten des mittleren Relativdrucks umfasst. Weiterhin kann die Steuereinrichtung eingerichtet sein, das Ausgleichsventil gemäß einer Vorgabe für den Relativdruck, einer Vorgabe für einen mittleren Entleerungsdruck und/oder einer Vorgabe für einen mittleren Füllungsdruck zu steuern. Die Steuereinheit kann auch eingerichtet sein, das Ausgleichsventil gemäß einer Relativdruckvorgabe und/oder einer Vorgabe für einen der beiden oder beide Umkehrpunkte zu steuern. Unter Steuern des Ausgleichsventils wird hier insbesondere ein Öffnen und Schließen des Ausgleichsventils zu bestimmten Zeiten verstanden. Weiterhin wird unter Steuern des Ausgleichsventils gemäß einer Relativdruck- oder Luftmassenvorgabe eine Regelung verstanden, in der beispielsweise eine Öffnungszeit des Ausgleichsventils als Stellgröße so eingestellt wird, dass einer Luftmassen- oder Relativdruckvorgabe als Regelgröße gefolgt werden kann.

Eine vorteilhafte Ausgestaltungsvariante der erfindungsgemäßen Antriebsvorrichtung sieht vor, dass die Steuereinheit eingerichtet ist, die Bewegung des Arbeitskolbens gemäß der zeitlichen Positionsvorgabe zu steuern und gleichzeitig das Ausgleichsventil gemäß der Vorgabe für den mittleren Relativdruck zu steuern.

Eine weitere vorteilhafte Ausgestaltungsvariante der erfindungsgemäßen Antriebsvorrichtung sieht vor, dass die Steuereinheit eingerichtet ist, die Bewegung des Arbeitskolbens einerseits gemäß der zeitlichen Relativdruckvorgabe oder gemäß der zeitlichen Kraftvorgabe zu steuern und andererseits gleichzeitig das Ausgleichsventil so zu steuern, dass das Totvolumen minimal ist, ohne dass der Arbeitskolben an der Wand des Hohlzylinders anschlägt. Das Totvolumen kann durch Vorgabe der Umkehrposition am Ende der Entleerungsphase in der Nähe der Wand klein gehalten werden.

Die Steuereinheit kann weiterhin eingerichtet sein, die zeitliche Positionsvorgabe basierend auf einer Referenztrajektorie für den Arbeitskolben zu bestimmen, wobei die Referenztrajektorie eine vorgegebene Dauer der ersten Phase, eine vorgegebene Dauer der zweiten Phase, eine vorgegebene Änderung der Beschleunigung des Kolbens in der ersten Phase, einen vorgegebenen Kolbenhub, einen vorgegebenen maximalen Relativdruck, eine vorgegebene Pumprate, eine vorgegebene relative Entleerungsdauer, einen vorgegebenen Füllungsgrad, einen vorgegebenen Entleerungsgrad, vorgegebene Kennwerte einer anzuschließenden Membranfluidpumpe, insbesondere ein Fördervolumen der Membranfluidpumpe, vorgegebene Kennwerte einer an die Membranfluidpumpe anschließbaren Einlasskanüle und/oder vorgegebene Kennwerte einer an die Membranfluidpumpe anschließbaren Auslasskanüle berücksichtigt.

Mithilfe der Referenztrajektorie können somit wichtige von dem Gesamtsystem einzuhaltende Randbedingungen vorgegeben werden, und die Positionsvorgabe kann unter der Erfüllung der Randbedingungen bestimmt werden. Das Totvolumen des Zylinders kann mithilfe der Positionsvorgabe minimal vorgegeben werden, um einen maximalen Wirkungsgrad der Arbeitspumpe zu erhalten. Durch Vorgabe des Kolbenhubs kann der Umkehrpunkt am Ende der Füllungsphase und damit auch ein Schlagvolumen, also das in einer Pumpzyklushälfte von dem Arbeitskolben durchlaufene Volumen eingestellt werden. Dadurch bewegt sich der Arbeitskolben innerhalb der eingestellten Umkehrpunkte. Besonders vorteilhaft ist es außerdem, wenn der vorgegebene Maximaldruck möglichst gering ist, um das durch die an die Antriebsvorrichtung anschließbare Membranfluidpumpe gepumpte Fluid nicht zu schädigen. Durch Angabe eines Entleerungs- und Füllungsgrad sowie der Pumprate kann der gewünschte von der Membranfluidpumpe zu pumpende Fluidfluss eingestellt bzw. an das mit der Membranfluidpumpe zu verbindende Fluidsystem angepasst werden. Ferner können die Übergangsphasen jeweils zwischen den drei Phasen so ausgelegt werden, dass eine ruckfreie und/oder belastungsarme Bewegung des Arbeitskolbens ermöglicht wird. Durch Angabe der Kennwerte für die Membranfluidpumpe und die Kanülen kann die Trajektorie an eine bestimmte Pumpen-Kanülen-Kombination angepasst werden.

Die Steuereinheit kann weiterhin eingerichtet sein, zum Steuern der Bewegung des Arbeitskolbens gemäß der zeitlichen Positionsvorgabe eine Antriebsstromstärke für den Spindelmotor basierend auf einem geschätzten erforderlichen Drehmoment für den Spindelmotor zu bestimmen, wobei in das Drehmoment eine Last des Arbeitskolbens, eine Trägheit des Arbeitskolbens, eine geschätzte Reibung des Arbeitskolbens und/oder eine Drehmomentkorrektur zur Kompensation einer Positionsabweichung und/oder einer Geschwindigkeitsabweichung des Arbeitskolbens von der zeitlichen Positionsvorgabe eingehen. Durch die Berücksichtigung der Last, der Trägheit und der Reibung des Arbeitskolbens kann eine höhere Positioniergenauigkeit erreicht werden.

Insbesondere kann die Steuereinrichtung eingerichtet sein, die Trägheit basierend auf einer aus der Referenztrajektorie resultierenden Beschleunigung zu bestimmen, die Last basierend auf einem gemessenen Relativdruck zu bestimmen und die Reibung basierend auf einer gemessenen Geschwindigkeit und/oder einer gemessenen Position abzuschätzen.

Die Antriebsvorrichtung kann weiterhin derart ausgebildet sein, dass während des Betriebs der Antriebsvorrichtung mittels eines Positionssensors eine momentane Position und/oder eine momentane Geschwindigkeit des Arbeitskolbens messbar sind. Die Steuereinheit kann dann eingerichtet sein, eine Positionsabweichung und/oder eine Geschwindigkeitsabweichung von der zeitlichen Positionsvorgabe zu bestimmen, basierend auf der Positionsabweichung und/oder der Geschwindigkeitsabweichung die Drehmomentkorrektur zu bestimmen und die Antriebsstromstärke derart anzupassen, dass die Positionsabweichung und/oder die Geschwindigkeitsabweichung des Arbeitskolbens von der zeitlichen Positionsvorgabe verringert werden. Auf diese Weise kann die Antriebsvorrichtung Positionsabweichungen des Arbeitskolbens kompensieren, und der Arbeitskolben dem gewünschten Verlauf folgen. Durch Überwachen des geschätzten Drehmoments durch die Reibung können während des Betriebs Verschleißerscheinungen des Arbeitskolbens erkannt werden. Die Antriebsstromstärke kann dann entsprechend korrigiert werden.

Die Gebrauchstauglichkeit der erfindungsgemäßen Antriebsvorrichtung wird erhöht, wenn die Antriebsvorrichtung eine Benutzerschnittstelle aufweist, mittels welcher benutzerspezifische Parameter, insbesondere ein Füllungs- und/oder Entleerungsgrad der Membranpumpe, ein Kolbenhub, ein mittlerer Relativdruck, eine relative Entleerungsdauer, ein Entleerungsdruck, ein Füllungsdruck und/oder eine Pumprate, während des Betriebs einstellbar sind. Die Steuereinheit kann dann eingerichtet sein, die zeitliche Positionsvorgabe, die zeitliche Relativdruckvorgabe und/oder die zeitliche Kraftvorgabe für die Bewegungssteuerung des Arbeitskolbens und/oder die Vorgabe für die Luftmasse, die Vorgabe für den Relativdruck und/oder die Vorgabe für die Luftmasse an einem der beiden oder an beiden Umkehrpunkten für die Steuerung des Ausgleichsventils während des Betriebs an eine Änderung der benutzerspezifischen Parameter anzupassen.

Weiterhin kann die Steuereinheit eingerichtet sein, einen Füllungsgrad der Membranpumpe durch Änderung eines Kolbenhubs und Steuern des Ausgleichsventils gemäß einer Vorgabe für den mittleren Entleerungsdruck im Arbeitsraum oder durch Änderung des mittleren Relativdrucks über das Ausgleichsventil einzustellen. Eine Erhöhung oder Verringerung des Füllungsgrades kann demnach entweder durch eine Erhöhung bzw. Verringerung des Kolbenhubs erfolgen, falls noch eine Hubreserve vorhanden ist, und konstant Halten des mittleren Entleerungsdrucks oder durch eine Absenkung bzw. Erhöhung des mittleren Relativdrucks, falls keine Hubreserve mehr vorhanden ist, erfolgen. Die Steuereinheit kann ebenso eingerichtet sein, einen Entleerungsgrad durch Änderung eines Kolbenhubs und Steuern des Ausgleichsventils gemäß einer Vorgabe für den mittleren Füllungsdruck im Arbeitsraum und/oder durch Änderung des mittleren Relativdrucks über das Ausgleichsventil einzustellen. Eine Erhöhung oder Verringerung des Entleerungsgrades kann demnach entweder durch eine Erhöhung bzw. Verringerung des Kolbenhubs erfolgen, falls noch eine Hubreserve vorhanden ist, und konstant Halten des mittleren Füllungsdrucks oder durch eine Erhöhung bzw. Absenkung des mittleren Relativdrucks, falls keine Hubreserve mehr vorhanden ist, erfolgen.

Die vorliegende Erfindung schließt auch ein Pumpensystem ein, welches eine Membranfluidpumpe, eine mit der Membranfluidpumpe verbundene Einlasskanüle zum Zuleiten eines Fluids zur Membranfluidpumpe sowie eine mit der Membranfluidpumpe verbundene Auslasskanüle zum Ableiten eines Fluids aus der Membranfluidpumpe sowie eine vorstehend beschriebene Antriebsvorrichtung für die Membranfluidpumpe umfasst.

Weiterhin schließt die vorliegende Erfindung ein Herzunterstützungssystem mit ein, welches eine Membranblutpumpe als Membranfluidpumpe, eine mit der Membranblutpumpe verbundene Einlasskanüle zum Zuleiten von Blut zur Membranblutpumpe aus einer Herzkammer und/oder einem Vorhof sowie eine mit der Membranblutpumpe verbundene Auslasskanüle zum Ableiten des Bluts aus der Membranblutpumpe in ein Blutgefäß sowie eine vorstehend beschriebene Antriebsvorrichtung für die Membranblutpumpe umfasst.

Erfindungsgemäß ist ferner ein Verfahren zum Betrieb der vorstehend beschrieben Antriebsvorrichtung für eine Membranfluidpumpe, wobei die Bewegung des Arbeitskolbens derart gesteuert wird, dass die Bewegung drei zeitlich aufeinanderfolgende Phasen umfasst, wobei in der ersten Phase der Arbeitskolben auf eine Geschwindigkeit beschleunigt wird, die größer ist als eine Geschwindigkeit am Ende der ersten Phase, in der zweiten Phase der Arbeitskolben derart bewegt wird, dass eine vorbestimmte Geschwindigkeit des Arbeitskolbens, ein vorbestimmter Relativdruck im Arbeitsraum oder eine vorbestimmte Kraft des Arbeitskolbens weitgehend konstant gehalten wird, und in der dritten Phase der Arbeitskolben mit einer weitgehend konstanten, negativen Beschleunigung bewegt wird.

Die Kraft kann insbesondere eine Last und eine Trägheit des Arbeitskolbens einschließen.

Das Verfahren kann weiterhin umfassen, dass die Bewegung des Arbeitskolbens derart gesteuert wird, dass der Arbeitskolben in der ersten Phase mit einer linear abnehmenden Beschleunigung bewegt wird.

Gemäß einer vorteilhaften Ausgestaltungsvariante des erfindungsgemäßen Verfahrens wird die Bewegung des Arbeitskolbens gemäß einer zeitlichen Positionsvorgabe für den Arbeitskolben gesteuert, wobei in der zweiten Phase die Geschwindigkeit des Arbeitskolbens weitgehend konstant gehalten wird.

Gemäß einer weiteren vorteilhaften Ausgestaltungvariante wird die Bewegung des Arbeitskolbens alternativ oder ergänzend zu der vorstehend genannten Ausgestaltungsvariante gemäß einer zeitlichen Relativdruckvorgabe für den Arbeitsraum gesteuert, wobei in der zweiten Phase der Relativdruck im Arbeitsraum weitgehend konstant gehalten wird.

Gemäß einer weiteren vorteilhaften Ausgestaltung wird die Bewegung des Arbeitskolbens alternativ oder ergänzend zu den beiden vorstehend genannten Ausgestaltungsvarianten gemäß einer zeitlichen Kraftvorgabe für den Arbeitskolben gesteuert, wobei in der zweiten Phase die Kraft des Arbeitskolbens weitgehend konstant gehalten wird.

Das Verfahren kann weiterhin umfassen, dass eine Steuerung gemäß der Positionsvorgabe, eine Steuerung gemäß der Relativdruckvorgabe und/oder eine Steuerung gemäß der Kraftvorgabe kombiniert werden. Beispielsweise kann das Verfahren umfassen, dass die Bewegung des Arbeitskolbens gesteuert wird, indem die zeitliche Positionsvorgabe basierend auf der zeitlichen Relativdruckvorgabe bestimmt wird, und die Bewegung des Arbeitskolbens gemäß der zeitlichen Positionsvorgabe gesteuert wird.

Als weitere Variante kann das Verfahren umfassen, dass die Bewegung des Arbeitskolbens in der ersten Phase und in der dritten Phase gemäß der zeitlichen Positionsvorgabe und in der zweiten Phase gemäß der zeitlichen Relativdruckvorgabe und/oder der zeitlichen Kraftvorgabe gesteuert wird. Als weitere Variante kann das Verfahren umfassen, dass die Positionsvorgabe basierend auf einer Verlaufsmessung des Relativdruckes aus vorhergehenden Pumpzyklen bestimmt wird.

Als weitere Variante kann das Verfahren umfassen, dass die zeitliche Kraftvorgabe basierend auf einer zeitlichen Positionsvorgabe bestimmt wird, und die Bewegung des Arbeitskolbens gemäß der Kraftvorgabe gesteuert wird. Beispielsweise kann mittels eines Positionssensors eine Differenz zwischen einem vorgegebenen Kolbenhub und einem erreichten Kolbenhub gemessen werden und die Kraft des Arbeitskolbens für den nächsten Pumpzyklus so angepasst werden, dass die Differenz im nächsten Pumpzyklus verringert wird.

Das Verfahren kann weiterhin umfassen, dass die Bewegung des Arbeitskolbens gemäß der zeitlichen Positionsvorgabe gesteuert wird und gleichzeitig das Ausgleichsventil gemäß einer Vorgabe für den mittleren Relativdruck gesteuert wird. Die Variante des Verfahrens ist wenig störanfällig und weist ein reproduzierbares Pumpverhalten auf, da zum einen die Positionsregelung und die Regelung des mittleren Relativdruckes zeitlich entkoppelt werden können, wodurch die Variante robust gegenüber Lastwechseln ist. Der Relativdruck kann bei steigenden Lasten erhöht werden. Eine Huberhöhung gleicht eine Empfindlichkeit gegenüber abnehmenden Lasten aus. Zum anderen kann die zu- oder abgeführte Luftmasse über die Messung des mittleren Relativdrucks besser kontrolliert werden, wodurch ein reproduzierbares und störunanfälliges vollständiges Füllen und Entleeren ermöglicht wird. Durch die Regelung des mittleren Relativdrucks können somit Störungen durch Leckage und Lastwechsel schnell erkannt und kompensiert werden.

Alternativ oder ergänzend kann das Verfahren umfassen, dass die Bewegung des Arbeitskolbens einerseits gemäß der zeitlichen Relativdruckvorgabe oder gemäß der zeitlichen Kraftvorgabe gesteuert wird und andererseits gleichzeitig das Ausgleichsventil so gesteuert wird, dass das Totvolumen minimal ist, ohne dass der Arbeitskolben an der Wand des Hohlzylinders anschlägt. Das Minimieren der Luftmasse am Umkehrpunkt am Ende der Entleerungsphase hat zwei Vorteile. Zum einen ist es bei der Steuerung gemäß der Relativdruck- oder Kraftvorgabe erforderlich die Umkehrpunkte vorzugeben, da andererseits Gefahr besteht, dass der Arbeitskolben gegen die Wand des Hohlzylinders läuft. Zum anderen ist der Wirkungsgrad der Arbeitspumpe größer, wenn das Totvolumen minimal ist. Eine Bewegungssteuerung gemäß der Relativdruckvorgabe hat den Vorteil, dass eine Reserve für Laständerungen getrennt für Füllen und Entleeren einstellbar ist. Eine Bewegungssteuerung gemäß der Kraftvorgabe hat den Vorteil, dass eine Reserve für Laständerungen getrennt für Füllen und Entleeren einstellbar ist und der Relativdruck bei steigenden Lasten erhöht wird.

Das Verfahren kann weiterhin umfassen, dass das Ausgleichsventil gemäß einer Vorgabe für einen mittleren Entleerungsdruck und/oder einer Vorgabe für einen mittleren Füllungsdruck unabhängig voneinander gesteuert wird.

Das Verfahren kann weiterhin umfassen, dass die zeitliche Positionsvorgabe basierend auf einer Referenztrajektorie für den Arbeitskolben bestimmt wird. Die Referenztrajektorie kann eine vorgegebene Dauer der ersten Phase, eine vorgegebene Dauer der zweiten Phase, eine vorgegebene Änderung der Beschleunigung des Arbeitskolbens in der ersten Phase, einen vorgegebenen Kolbenhub, einen vorgegebenen maximalen Relativdruck, eine vorgegebene Pumprate, eine vorgegebene relative Entleerungsdauer, einen vorgegebenen Füllungsgrad, einen vorgegebenen Entleerungsgrad, vorgegebene Kennwerte einer anzuschließenden Membranfluidpumpe, insbesondere ein Fördervolumen der Membranfluidpumpe, vorgegebene Kennwerte einer an die Membranfluidpumpe anschließbaren Einlasskanüle und/oder vorgegebene Kennwerte einer an die Membranfluidpumpe anschließbaren Auslasskanüle berücksichtigen.

Das Verfahren kann weiterhin umfassen, dass zum Steuern der Bewegung des Arbeitskolbens gemäß der Positionsvorgabe eine Antriebsstromstärke für den Spindelmotor bestimmt wird. Die Bestimmung der Antriebsstromstärke kann auf einem geschätzten erforderlichen Drehmoment für den Spindelmotor basieren. In das Drehmoment können eine Last des Arbeitskolbens, eine Trägheit des Arbeitskolbens, eine geschätzte Reibung des Arbeitskolbens und/oder eine Drehmomentkorrektur zur Kompensation einer Positionsabweichung und/oder einer Geschwindigkeitsabweichung des Arbeitskolbens von der zeitlichen Positionsvorgabe eingehen. Die Reibung schließt insbesondere eine Haft- und Gleitreibung ein. Durch Berücksichtigung der Reibung können Störungen durch mechanischen Verschleiß schneller erkannt und kompensiert werden.

Insbesondere kann das Verfahren umfassen, dass die Trägheit basierend auf einer aus der Referenztrajektorie resultierenden Beschleunigung bestimmt wird, die Last basierend auf einem gemessenen Relativdruck bestimmt wird, und die Reibung basierend auf einer gemessenen Geschwindigkeit und/oder einer gemessenen Geschwindigkeit abgeschätzt wird.

Das Verfahren kann weiterhin umfassen, dass mittels eines Positionssensors eine momentane Position und/oder eine momentane Geschwindigkeit des Arbeitskolbens während des Betriebs gemessen werden. Das Verfahren kann weiterhin umfassen, dass eine Positionsabweichung und/oder Geschwindigkeitsabweichung von der zeitlichen Positionsvorgabe bestimmt wird, basierend auf der Positionsabweichung und/oder der Geschwindigkeitsabweichung die Drehmomentkorrektur bestimmt wird und die Antriebsstromstärke derart angepasst wird, dass die Positionsabweichung und/oder die Geschwindigkeitsabweichung des Arbeitskolbens von der zeitlichen Positionsvorgabe verringert werden.

Das Verfahren kann weiterhin umfassen, dass benutzerspezifische Parameter, insbesondere ein Füllungs- und/oder Entleerungsgrad der Membranpumpe, ein Kolbenhub, ein mittlerer Relativdruck, eine relative Entleerungsdauer, ein Entleerungsdruck, ein Füllungsdruck und/oder eine Pumprate, während des Betriebs eingestellt werden. Das Verfahren kann weiterhin umfassen, dass die zeitliche Positionsvorgabe, die zeitliche Relativdruckvorgabe und/oder die zeitliche Kraftvorgabe für die Bewegungssteuerung des Arbeitskolbens und/oder die Vorgabe für die Luftmasse und/oder die Vorgabe für den Relativdruck für die Steuerung des Ausgleichsventils während des Betriebs an eine Änderung der benutzerspezifischen Parameter angepasst werden.

Das Verfahren kann weiterhin umfassen, dass ein vorgegebener Füllungsgrad der Membranfluidpumpe durch Änderung eines Kolbenhubs und Steuern des Ausgleichsventils gemäß einer Vorgabe für einen mittleren Entleerungsdruck im Arbeitsraum oder durch Änderung des mittleren Relativdruckes über das Ausgleichsventil eingestellt wird. Insbesondere kann das Verfahren umfassen, dass der Füllungsgrad erhöht oder verringert wird, indem der Kolbenhub erhöht bzw. verringert wird, falls noch eine Hubreserve vorhanden ist, und der mittlere Entleerungsdruck konstant gehalten wird, oder indem der mittlere Relativdruck abgesenkt bzw. erhöht wird, falls keine Hubreserve mehr vorhanden ist.

Das Verfahren kann weiterhin umfassen, dass ein vorgegebener Entleerungsgrad durch Änderung eines Kolbenhubs und Steuern des Ausgleichsventils gemäß einer Vorgabe für einen mittleren Füllungsdruck im Arbeitsraum und/oder durch Änderung des mittleren Relativdruckes über das Ausgleichsventil eingestellt wird. Insbesondere kann das Verfahren umfassen, dass der Entleerungsgrad erhöht oder verringert wird, indem der Kolbenhub erhöht bzw. verringert wird, falls noch eine Hubreserve vorhanden ist, und der mittlere Füllungsdruck konstant gehalten wird, oder indem der mittlere Relativdruck erhöht bzw. abgesenkt wird, falls keine Hubreserve mehr vorhanden ist.

Im Folgenden werden eine erfindungsgemäße Antriebsvorrichtung, ein erfindungsgemäßes Herzunterstützungssystem sowie ein Verfahren zum Betrieb der Antriebsvorrichtung anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des jeweiligen Beispiels und weiterer Merkmale des jeweiligen Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: eine schematische Darstellung eines erfindungsgemäßes Herzunterstützungssystems,
- Figur 2: ein Beschleunigung-Zeit-Diagramm mit einem schematisch dargestellten Beschleunigungsverlauf für den Arbeitskolben einer erfindungsgemäßen Antriebsvorrichtung während einer Pumpzyklushälfte,
- Figur 3: ein Schema zur Bestimmung der Positionsvorgabe, der Positionsregelung und der Regelung für den mittleren Relativdruck gemäß der Erfindung und
- Figur 4: Verlaufsdiagramme für einen Pumpzyklus einer Menge von Zustandsgrößen, welche ein konkretes Bewegungsbeispiel des Arbeitskolbens zeigen.

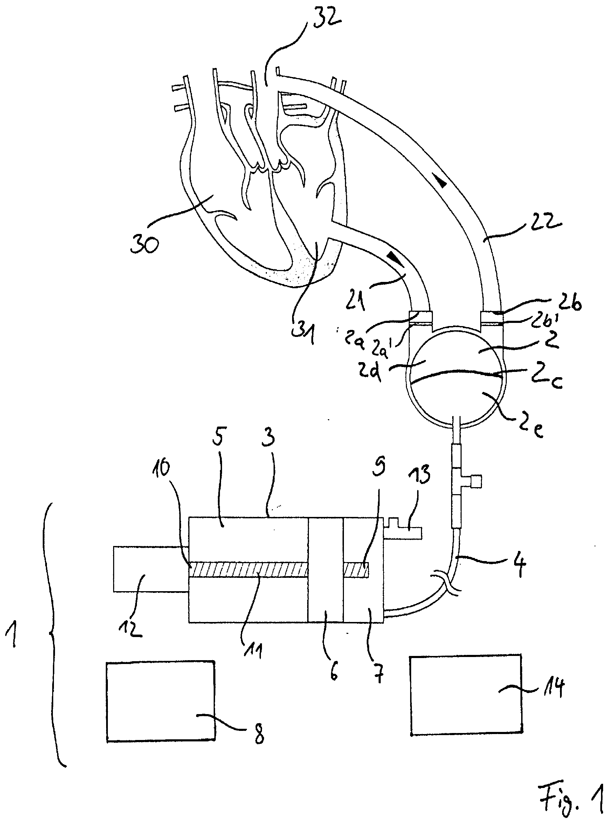

Figur 1 zeigt eine schematische Darstellung eines erfindungsgemäßen Herzunterstützungssystems für ein menschliches Herz 30. Das erfindungsgemäße Herzunterstützungssystem weist eine extrakorporale Membranblutpumpe 2 sowie eine erfindungsgemäße Antriebsvorrichtung 1 auf. Die Antriebsvorrichtung 1 und die Membranblutpumpe 2 sind über eine Druckleitung 4 miteinander verbunden. Die Membranblutpumpe 2 weist einen Einlass 2a und einen Auslass 2b auf. Am Einlass 2a ist die Membranblutpumpe 2 über eine Einlasskanüle 21 mit einer linken Herzkammer 31 des Herzens 30 verbunden. Am Auslass 2b ist die Membranblutpumpe 2 über eine Auslasskanüle mit einem Blutgefäß (Aorta) 32 verbunden. Weiterhin umfasst die Membranblutpumpe 2 eine Blutkammer 2d und eine Luftkammer 2e, die durch eine Membran 2c voneinander getrennt sind. Die Blutkammer 2d steht dabei in einer Stoffaustauschverbindung und Druckaustauschverbindung mit der Ein- und Auslasskanüle 21 und 22 sowie mit linken Herzkammer 31 und der Aorta 32, das heißt, das Blut kann aus der linken Herzkammer 31 in die Blutkammer 2d und von dort in die Aorta 32 fließen. Die Luftkammer 2e steht in einer Stoffaustauschverbindung und Druckaustauschverbindung mit der Druckleitung 4 und der Antriebsvorrichtung 1, das heißt, Luft aus der Antriebsvorrichtung 1 und der Druckleitung 4 strömt auch in die Luftkammer 2e. Über die Membran 2c der Membranblutpumpe 2 kann ein Relativdruck von der Luftkammer 2e auf die Blutkammer 2d, und somit von der Antriebsvorrichtung 1 auf die Blutkammer 2d übertragen werden. Unter Relativdruck wird hier ein Luftdruck in der Druckleitung 4 relativ zu einem Umgebungsdruck verstanden.

Die Antriebsvorrichtung 1 weist einen Pneumatik-Zylinder 5 mit einem hohlzylinderförmigen Gehäuse 5 und einem darin oszillatorisch zwischen zwei Umkehrpunkten 9 und 10 hin und her bewegbaren Arbeitskolben 6 auf. Die Umkehrpunkte 9 und 10 stellen keine Fixpunkte dar, sondern können gezielt eingestellt werden. Der Pneumatik-Zylinder ist an einer seiner Stirnseiten mit der Druckleitung 4 verbunden. Zur Druckleitung 4 hin schließt der Arbeitskolben 6 einen Arbeitsraum 7 mit dem Gehäuse 5 ein. Der Arbeitsraum 7 steht in einer Stoffaustauschverbindung und Druckaustauschverbindung mit der Druckleitung 4. Im Bereich des Arbeitsraumes 7 weist der Pneumatik-Zylinder 3 weiterhin ein Ausgleichsventil 13 zum Ausgleichen einer Luftmasse und/oder eines Relativdrucks im Arbeitsraum 7 mit der Umgebung auf. Der Arbeitskolben 6 ist über eine Spindel 11 eines elektrischen Spindelmotors 12 bewegbar. Des Weiteren weist die Antriebsvorrichtung 1 eine Steuereinheit 8 zum Steuern der Bewegung des Arbeitskolbens 6 und zum Steuern des Ausgleichsventils 13 sowie eine Benutzerschnittstelle 14 zum Einstellen von benutzerspezifischen Parametern auf.

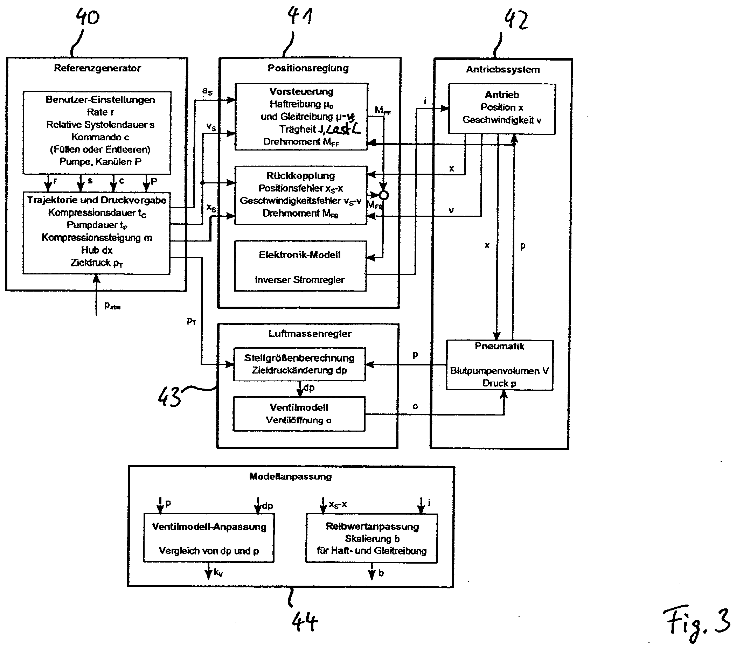

Für den Betrieb der Antriebsvorrichtung gibt die Steuereinheit 8 den Strom für den Spindelmotor 12 vor. Wie der Strom konkret bestimmt wird, wird weiter unten anhand von Figur 3 erläutert. Der Spindelmotor 12 treibt die Spindel 11 an. Dabei wird eine Rotationsbewegung des Motors 12 über die Spindel 11 in eine translatorische Bewegung des Arbeitskolbens 6 übertragen, wodurch sich der Arbeitskolben 6 innerhalb des Gehäuses 5 zwischen den Umkehrpunkten 10 und 9 hin und her bewegt. Bewegt sich der Arbeitskolben 6 zu dem an der Druckleitung 4 gelegen Umkehrpunkt 9 wird die Luft im Arbeitsraum 7 und in der Druckleitung komprimiert. Der im Arbeitsraum 7, in der Druckleitung 4 und in der Luftkammer 2e vorliegende Relativdruck wird auch auf die Membran 2c übertragen. Ab einem bestimmten Relativdruck beginnt die Membran 2c sich in Richtung der Kanülen 21 und 22 vorzuwölben und ihrerseits einen Druck auf das sich in der Blutkammer 2d befindliche Blut aufzubauen. Am Einlass 2a und am Auslass 2b sind Ventile 2a' bzw. 2b' angeordnet. In der Entleerungsphase ist das Ventil 2a' am Einlass 2a geschlossen und das Ventil 2b' am Auslass 2b geöffnet, so dass das sich in der Blutkammer 2d befindliche Blut durch das Vorwölben der Membran 2c durch den Auslass 2b in die Auslasskanüle 22 und von dort in das Blutgefäß 32 gedrückt werden kann. Optimal ist es, wenn sich die Membran 2c bei Erreichen des Umkehrpunktes 9 vollständig in Richtung der Kanülen 21, 22 entfaltet hat, jedoch nicht überdehnt ist. In diesem Fall wird die Blutkammer vollständig entleert, ohne die Membran 2c zu schädigen.

Nach Erreichen des Umkehrpunktes 9 bewegt sich der Arbeitskolben 6 in die entgegengesetzte Richtung zum Umkehrpunkt 10. Dadurch nimmt der Relativdruck im Arbeitsraum 7 rasch ab. Ab einem bestimmten negativen Druck beginnt die Membran 2c der Membranblutpumpe 2 sich zur Druckleitung hin und von den Kanülen 21 und 22 weg zu wölben. Dadurch wird ein Saugdruck auf die Blutkammer 2d ausgeübt. In der Füllungsphase ist das Ventil 2a' am Einlass 2a geöffnet und das Ventil 2b' am Auslass 2b geschlossen. Durch den Saugdruck wird nun Blut aus der Herzkammer 31 durch die Einlasskanüle 21 in die Blutkammer 2d gesaugt. Optimal ist es, wenn sich die Membran bei Erreichen des Umkehrpunktes 10 vollständig in Richtung der Druckleitung 4 entfaltet hat, jedoch nicht überdehnt ist. In diesem Fall wird die Blutkammer 2d vollständig gefüllt, ohne die Membran 2c zu schädigen.

Über die Benutzerschnittstelle 14 kann das Fördervolumen der Membranblutpumpe 2 eingestellt werden. Weiterhin können der Füllungs- und Entleerungsgrad der Membranblutpumpe 2 unabhängig voneinander angepasst werden, falls sich während des Betriebs das Verhalten der Membran 2c ändert bzw. die Membran 2c nicht mehr das oben beschriebene optimale Verhalten zeigt oder sich in Füllungs- und Entleerungsphase unterschiedliche Abweichungen vom optimalen Verhalten ergeben. Die Einstellung der Membranblutpumpe mithilfe der Benutzerschnittstelle 14 erfolgt dabei anhand einer visuellen Prüfung der Membranbewegung. Dabei können insbesondere hohe Relativdrücke vermieden werden, indem die Intensität der Einstellung möglichst gering gehalten wird.

Die Bewegung des Arbeitskolbens wird durch die Steuereinheit so gesteuert, dass sich eine gegenüber dem Stand der Technik eine erhöhte Pumpleistung, ergibt. Während des Betriebs der Antriebsvorrichtung werden daher die Position des Arbeitskolbens 6, der Relativdruck im Arbeitsraum 7 und die Kraft des Arbeitskolbens 6 ständig überwacht und Abweichungen von einer Vorgabe durch eine gezielte Steuerung korrigiert.

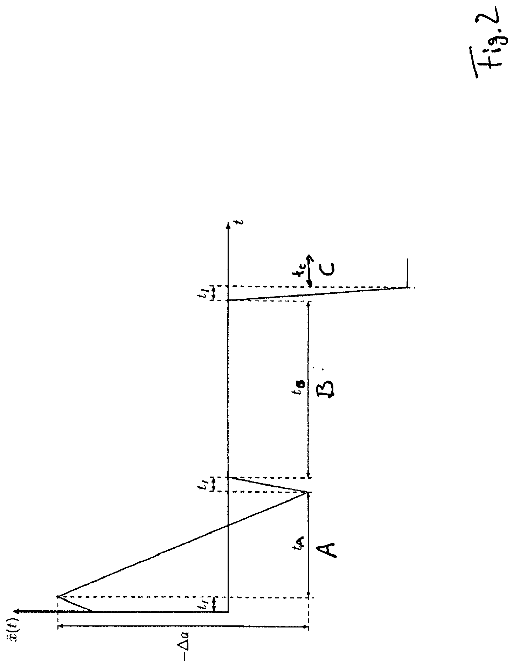

Ein Ausführungsbeispiel sieht vor, dass die Bewegung des Arbeitskolbens 6 gemäß einer Positionsvorgabe gesteuert wird und gleichzeitig der Mitteldruck im Arbeitsraum 7 konstant gehalten wird. Eine optimale Positionsvorgabe ergibt sich bei Einhaltung eines zeitlichen Beschleunigungsverlaufs in einer Pumpzyklushälfte gemäß Figur 2. Wie die konkrete Positionsvorgabe und der daraus resultierende Strom für den Spindelmotor 12 bestimmt wird, wird anhand von Figur 3 erläutert. Der in Figur dargestellte Beschleunigungsverlauf ermöglicht eine hohe Pumpleistung und einen schonenden Betrieb durch gleichmäßiges Pumpen der Membranblutpumpe 2 und eine ruckfreie Bewegung des Arbeitskolbens 6 sowie eine Verringerung der Gefahr der Blutschädigung. Der Beschleunigungsverlauf weist im Wesentlichen drei Phasen A, B, C mit Zeitdauern t_{A}, t_{B} und t_{C} auf, wobei der Arbeitskolben 6 in der ersten Phase A mit einer linear abnehmenden Beschleunigung bewegt wird, in der zweiten Phase B unbeschleunigt, d.h. mit konstanter Geschwindigkeit, bewegt wird und in der dritten Phase C mit einer konstanten, negativen Beschleunigung abgebremst wird. Jeweils zwischen zwei der drei Phasen A, B, C befindet sich eine Übergangsphase mit Zeitdauer t_{I}, welche jeweils zwei der drei Phasen A,B, C des Beschleunigungsverlaufs stetig miteinander verbinden, um eine ruckfreie und/oder belastungsarme Bewegung des Arbeitskolbens zwischen den Phasen A, B und C zu ermöglichen.

Während des Betriebs wird nun die momentane Position und Geschwindigkeit des Arbeitskolbens 6 alle 1-10 ms, insbesondere alle 2 ms, mittels eines Positionssensors gemessen und eine Einhaltung der Positionsvorgabe überprüft. Dazu weist die Arbeitspumpe 3 einen Zähler für Motor-Hall-Sensor-Inkremente (hier nicht dargestellt) und mindestens einen zusätzlichen Hall-Sensor und/oder einen mechanischen Anschlag (beide hier nicht gezeigt) am Arbeitskolben 6 auf, der an einer festen Referenzposition anspricht. Über den zweiten Hall-Sensor oder den mechanischen Anschlag kann der Zähler bei der Inbetriebnahme zurückgesetzt werden. Eine Geschwindigkeit des Arbeitskolbens 6 wird von der Steuereinheit 8 direkt aus der Zeitmessung zwischen zwei Inkrementen bestimmt. Die Steuereinheit 8 bestimmt eine Abweichung von der Positionsvorgabe und passt auf Grundlage dieser Abweichung den dem Spindelmotor 12 zugeführten Strom so an, dass die Abweichung verringert wird. Weiterhin wird alle 2ms der Relativdruck gemessen und ein momentaner mittlerer Relativdruck über den vergangenen Pumpzyklus bestimmt. Die Steuereinheit 8 bestimmt eine Abweichung von dem vorgegebenen mittleren Relativdruck und steuert das Ausgleichsventil 13 so, dass die Abweichung von dem vorgegebenen mittleren Relativdruck verringert wird. Dies geschieht, in dem sowohl in der Entleerungsphase als auch in der Füllungsphase das Ausgleichsventil 13 für eine bestimmte Zeit geöffnet wird. Die Öffnungsdauer des Ausgleichsventils 13 wird bestimmt, indem drei Bereiche für Mitteldruckabweichungen unterschieden werden und diesen gewünschte Mitteldruckänderungen zugeordnet werden. Je größer dabei die mittlere Abweichung in dem jeweiligen Bereich ist, desto größer ist die Mitteldruckänderung. In Abhängigkeit vom Betrag des gemessenen Relativdrucks (und vom geschätzten Volumen) wird die nötige Öffnungsdauer berechnet.

Ist der mittlere Relativdruck zu hoch, wird das Ausgleichsventil 13 in der darauffolgenden Entleerungsphase geöffnet, um Luft abzuführen. Ist der mittlere Relativdruck zu niedrig, wird das Ausgleichsventil 13 in der darauffolgenden Füllungsphase geöffnet, um Luft zuzuführen.

Ein weiteres Ausführungsbeispiel sieht vor, die Bewegung des Arbeitskolbens 6 gemäß einer Relativdruckvorgabe zu steuern und das Totvolumen des Zylinders mithilfe des Ausgleichsventils 13 zu minimieren und konstant zu halten. In der zweiten Phase wird der Arbeitskolben dabei so bewegt, dass der Relativdruck konstant gehalten wird. Während des Betriebs wird alle 1-10 ms, insbesondere alle 2 ms, der momentane Relativdruck im Arbeitsraum 7 gemessen. Die Steuereinheit 8 bestimmt eine Abweichung des momentanen Relativdrucks von der Vorgabe und passt die Stromstärke für den Spindelmotor 12 so an, dass die Abweichung von der Relativdruckvorgabe im weiteren Bewegungsverlauf des Arbeitskolbens 6 verringert wird. Zusätzlich wird ständig die Position des Arbeitskolbens 6 mittels eines Positionssensors überwacht, wobei die Steuereinheit 8 überprüft, ob eine gewünschte Endposition des Arbeitskolbens 6 erreicht wird. Wenn dies nicht der Fall ist, wird je nach überschreiten oder unterschreiten der gewünschten Endposition in der Füllungsphase oder in der Entleerungsphase einmal für eine gewisse Zeit das Ausgleichsventil 13 geöffnet, um dem Arbeitsraum 7 Luft zuzuführen oder Luft abzuführen. Je nach Größe der Abweichung von der vorbestimmten Endposition wird das Ausgleichsventil 13 länger oder kürzer geöffnet.

Ein weiteres Ausführungsbeispiel sieht vor, dass die oben beschriebene Relativdruckregelung von einer Drehzahl- oder Positionsregelung unterlagert wird, da in diesem Beispiel eine reine Druckregelung gegenüber Laständerungen oder Leckage zu störanfällig ist. In diesem Fall wird die Stromanpassung aufgrund einer Druckabweichung von der Relativdruckvorgabe im Rahmen der Drehzahl- bzw. Positionsvorgabe vorgenommen. Das heißt, die Druckänderungsvorgabe geht zunächst in die Drehzahl- bzw. Positionsvorgabe ein. Aufgrund der Druckänderungsvorgabe bestimmt die Steuereinheit somit zunächst eine Drehzahl- bzw. Positionsänderungsvorgabe, auf Basis derer die Stromanpassung berechnet wird. Insbesondere durch die unterlagerte Drehzahlregelung lässt sich auf einfache Weise eine Maximalgrenze für die Drehzahl einhalten, die im Betrieb nicht überschritten werden darf.

Ein weiteres Ausführungsbeispiel sieht vor, dass die Steuereinheit die Bewegung des Arbeitskolbens 6 gemäß einer Kraftvorgabe für den Arbeitskolben 6 steuert, wobei in der zweiten Phase die Summe aus Trägheitskraft des Arbeitskolbens 6 und Last des Arbeitskolbens 6 konstant gehalten wird. Die Kraftvorgabe für den Arbeitskolben 6 wird über die kinematischen Beziehungen zwischen dem Arbeitskolben 6 und dem Spindelmotor 12 in eine Drehmomentvorgabe für den Spindelmotor 12 umgerechnet. Während des Betriebs wird das Drehmoment des Spindelmotors 12, welches die Trägheitskraft des Arbeitskolbens 6 und die Last des Arbeitskolbens überwindet, ständig überwacht. Hierzu wird alle 1-10 ms, insbesondere alle 2 ms, über den Positionssensor die momentane Position und die momentane Geschwindigkeit sowie über den Drucksensor der momentane Relativdruck gemessen. Die Steuereinheit 8 bestimmt aus diesen Werten das momentane Drehmoment und bestimmt eine Abweichung des momentanen Drehmoments von der Vorgabe. Die Steuereinheit 8 passt den Strom so an, dass die Abweichung verringert wird. Gleichzeitig hält die Steuereinheit mithilfe des Ausgleichsventils 13 wie bei der Druckregelung die Luftmasse in der Endposition in der Entleerungsphase möglichst klein, indem bei einer Druckabweichung einmal pro Pumpzyklus das Ausgleichsventil 13 für eine entsprechende Zeit geöffnet wird. Auch bei der Kraftregelung ist es notwendig die Endpositionen des Arbeitskolbens 6 gesondert vorzugeben, da sonst der Arbeitskolben 6 gegen die Gehäusewand laufen würde. Das Erreichen jeder Endposition wird mittels eines Positionssensors überwacht. Dieser misst während des Betriebs, ob die jeweilige Endposition erreicht wird. Sollten beide Endpositionen nicht erreicht werden, kann die Kraft durch Anpassen der Stromstärke entsprechend angepasst werden. Sollte ein Abstand des Arbeitskolbens 6 zur Gehäusewand zu gering werden, muss andererseits sofort die Bewegungsrichtung des Arbeitskolbens 6 geändert werden.

Über die Benutzerschnittstelle 14 kann die Menge des durch die Membranblutpumpe geförderten Blutes (Fördervolumen) eingestellt werden. Ferner können der Füllungs- und Entleerungsgrad getrennt voneinander eingestellt werden. Dies soll nachfolgend am Beispiel der Positionsregelung erklärt werden. Soll beispielsweise der Füllungsgrad erhöht oder verringert werden, so wird der Kolbenhub in der Füllungsphase erhöht bzw. verringert, sofern noch eine Hubreserve zur Verfügung steht, und von einer Regelung des mittleren Relativdrucks auf eine Regelung des mittleren Entleerungsdrucks mithilfe des Ausgleichsventils 13 umgestellt. Sofern keine weiteren Kolbenhubänderungen vorgenommen worden sind, wird nach einer vorbestimmten Anzahl an Pumpzyklen nach der letzten Änderung des Füllungs- und/oder Entleerungsgrads auf eine Regelung des mittleren Relativdrucks zurückgestellt. Die vorbestimmte Anzahl an Pumpzyklen liegt vorzugsweise zwischen 20 und 100, insbesondere bei 50. Sollte jedoch kein Kolbenhub mehr zur Verfügung stehen, wird der mittlere Relativdruck abgesenkt bzw. erhöht. Analog wird der Entleerungsgrad geändert. Soll der Entleerungsgrad erhöht oder verringert werden, so wird der Kolbenhub in der Entleerungsphase erhöht bzw. verringert, sofern noch eine Hubreserve zur Verfügung steht, und von einer Regelung des mittleren Relativdrucks auf eine Regelung des mittleren Füllungsdrucks mithilfe des Ausgleichsventils 13 umgestellt. Sofern keine weiteren Kolbenhubänderungen vorgenommen worden sind, wird nach einer vorbestimmten Anzahl an Pumpzyklen auf eine Regelung des mittleren Relativdrucks zurückgestellt. Die vorbestimmte Anzahl an Pumpzyklen liegt vorzugsweise zwischen 20 und 100, insbesondere bei 50. Sollte jedoch kein Kolbenhub mehr zur Verfügung stehen, wird der mittlere Relativdruck erhöht bzw. abgesenkt.

Bei der Relativdruckregelung wird die Pumprate auf Grund des benötigten Fluidflusses eingestellt. Über die Füllungs- und Entleerungsdrücke wird das Füllungs- und Entleerungsverhalten eingestellt. Um den Betrag der Füllungsdrücke abzusenken, kann der Benutzer die Entleerungsdauer verlängern.

Figur 3 zeigt ein Schema zur Bestimmung der Positionsvorgabe, der Positionsregelung und der Regelung für den mittleren Relativdruck gemäß der Erfindung. In einem Referenzgenerator 40 wird zunächst eine Referenztrajektorie ermittelt und ein mittlerer Relativdruck vorgegeben. Ausgangspunkt für die Referenztrajektorie ist ein qualitativer zeitlicher Verlauf der Geschwindigkeit und der Beschleunigung, der durch die Vorgabe der ersten bis dritten Phase charakterisiert ist. In Figur 2 ist beispielsweise der qualitative zeitliche Verlauf der Beschleunigung gezeigt. Der qualitative Beschleunigungsverlauf wird dann durch die konkrete Wahl der Trajektorienparameter Kompressionsdauer t_{C}, der Pumpdauer t_{P}, der Kompressionssteigung m und des Kolbenhubs dx, an eine konkrete Membranfluidpumpen-Kanülen-Kombination P angepasst. Diese Referenztrajektorie wird anschließend durch die Wahl von bestimmten Benutzer-Einstellungen skaliert. Die Benutzer-Einstellungen können eine Pumprate r, eine relative Entleerungsdauer s (Dauer der Entleerungsphase in Bezug auf eine Pumpzyklushälfte) und Änderungsvorgaben für einen Füllungs- und Entleerungsgrad sein. Die Pumprate r wird je nach erforderlichem Blutfluss eingestellt. Die relative Entleerungsdauer wird im vorliegenden Beispiel vorzugsweise auf etwa 50% gestellt. Das Einstellen von Füllen und Entleeren dient insbesondere zum Beschränken des Füllungs- bzw. Entleerungsdrucks. Die Benutzer-Einstellungen werden in der Steuereinheit in die Trajektorien-Parameter umgerechnet, und die Trajektorien-Parameter entsprechend angepasst, wodurch sich eine angepasste Referenztrajektorie ergibt. Die Referenztrajektorie liefert eine Vorgabe für die Soll-Beschleunigung a_{S}, die Soll-Geschwindigkeit v_{S} und die Soll-Position x_{S}.

Die Vorgaben der Referenztrajektorie werden an die Positionsregelung 41 übergeben. Die Soll-Beschleunigung und die Soll-Geschwindigkeit werden in der Vorsteuerung zur Bestimmung des Drehmoments M_{FF} für den Spindelmotor 12 zur Überwindung der Last L, der Trägheit J und der Reibung µ₀, µ*vₛ des Arbeitskolbens verwendet. Dabei wird die Reibung µ₀, µ*vₛ mithilfe eines Modells für ein Gesamtreibmoment b (µ₀ + µ*vₛ) abgeschätzt. In dem Modell setzt sich das Gesamtreibmoment aus einer Summe aus Haftreibmoment µ₀ und Gleitreibmoment µ*vₛ zusammen, wobei die Summe anschließend mit einem Faktor b skaliert wird. Über den einzelnen Faktor b kann das Gesamtreibmoment für die Positionsvorgabe angepasst werden. Das Reibmoment stellt einen zeitlich veränderbaren Modellparameter dar. Die Änderung des Reibmoments resultiert beispielsweise aus einer Alterung des Pneumatik-Zylinders und kurzfristigen Einflüssen, wie z.B. Temperaturschwankungen.

Die Soll-Geschwindigkeit und die Soll-Position werden außerdem an die Rückkopplung übergeben. Im Antriebssystem (Pneumatik-Zylinder) 42 werden die Ist-Position x und Ist-Geschwindigkeit v gemessen. Diese werden dann ebenfalls an die Rückkopplung übergeben. In der Rückkopplung werden die Differenzen x_{S}-x zwischen der Soll-Position und der Ist-Position und v_{S}-v zwischen der Soll-Geschwindigkeit und der Ist-Geschwindigkeit bestimmt. Aufgrund der Positions- und Geschwindigkeitsdifferenzen wird eine Drehmomentkorrektur M_{FB} bestimmt.

Die Drehmomente M_{FF} und M_{FB} werden dann addiert und über ein Elektronik-Modell mit einem inversen Stromregler in einen Strom i umgerechnet. Der Strom i wird dem Antriebssystem zugeführt.

Die Zieldruckvorgabe p_{T} aus dem Referenzgenerator kann beispielsweise eine Vorgabe für den mittleren Relativdruck sein, der konstant gehalten werden soll. Die Zieldruckvorgabe wird an den Luftmassenregler 43 übergeben. Im Antriebssystem (Pneumatik-Zylinder) wird ein Ist-Druck gemessen. Der Ist-Druck wird an den Luftmassenregler 43 übergeben und eine Druck-Differenz berechnet. Aufgrund der Druck-Differenz wird eine Zieldruckänderungsvorgabe dp an ein Ventilmodell für das Ausgleichsventil 13 übergeben. Das Ventilmodell rechnet die Zieldruckänderungsvorgabe in eine Ventilöffnungszeit o um und gibt diese an das Ausgleichsventil 13 im Antriebssystem aus.

Der positionsgeregelte Antrieb im Antriebssystem 42 stellt zu jedem Zeitpunkt die Position x des Arbeitskolbens ein. Der momentan gemessene Relativdruck p ist die Reaktion des angeschlossenen Systems auf die Position x. Der Relativdruck wird von der Luftmasse, dem Volumen und den Lasten beeinflusst.

Das Ventilmodell und das Reibwertmodell können im Verlauf der Regelung durch eine Modellanpassung 44 automatisch angepasst werden. Durch einen Vergleich der Zieldruckänderungsvorgabe dp mit dem nachfolgend gemessenen Druck p kann eine Ventilkonstant k_{V} so angepasst werden, dass im Verlauf der Regelung die Zieldruckänderungsvorgabe besser eingehalten wird. Die Reibwertanpassung erfolgt, wenn die Differenz zwischen Soll- und Ist-Position einen bestimmten Wert überschreitet und für eine bestimmte Zeit bestehen bleibt sowie, wenn sich der Strom i in einem bestimmten Bereich bewegt. Das Gesamtreibmoment wird über eine Änderung des Skalierungsfaktors b angepasst.

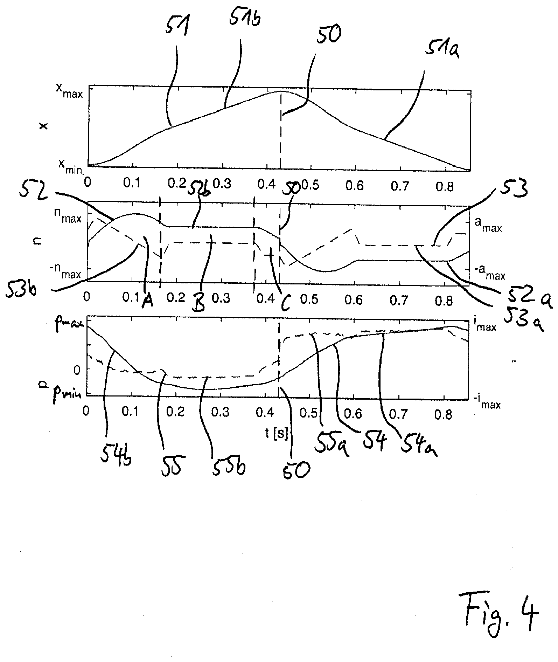

Figur 4 zeigt für einen Pumpzyklus Verlaufsdiagramme von Zustandsgrößen, welche einen qualitativen zeitlichen Verlauf des Zustands des Arbeitskolbens 6 beschreiben. Die Verlaufsdiagramme zeigen jeweils eine Füllungs- und eine Entleerungsphase, welche durch eine gestrichelte Linie 50 optisch voneinander getrennt sind. Die Kurve 51 zeigt einen zeitlichen Positionsverlauf. Der Abschnitt 51a zeigt dabei die Entleerungsphase, während der Abschnitt 51b die Füllungsphase zeigt. Gemäß der Kurve 50 bewegt sich der Arbeitskolben 6 von der Endposition einer vorhergehenden Entleerungsphase bei x = xₘᵢₙ zur Endposition der Füllungsphase 51b bei x = xₘₐₓ und von dort zurück zur Endposition der Entleerungsphase 51a bei x = xₘᵢₙ mm.

Die Kurve 52 zeigt einen Drehzahlverlauf bzw. Geschwindigkeitsverlauf, wobei der Abschnitt 52a die Entleerungsphase und der Abschnitt 52b die Füllungsphase zeigt. Die Drehzahl des Spindelmotors 12 lässt sich über die kinematischen Beziehungen zwischen dem Spindelmotor 12 und dem Arbeitskolben 6 in eine Geschwindigkeit des Arbeitskolbens 6 umrechnen. Die Drehzahl bzw. Geschwindigkeit des Arbeitskolbens 6 steigt im ersten Teil der Füllungsphase 52b, welcher der ersten Phase A entspricht, zunächst vom Stillstand des Arbeitskolbens 6 auf eine Geschwindigkeit an, die größer ist als eine Geschwindigkeit des Arbeitskolbens 6 am Ende der ersten Phase A. Im mittleren Teil der Füllungsphase 52b, welcher der zweiten Phase B entspricht, bleibt die Geschwindigkeit weitgehend konstant. Im letzten Teil der Füllungsphase 52b, welcher der dritten Phase C entspricht, nimmt die Geschwindigkeit weitgehend linear ab. Die Entleerungsphase 52a zeigt einen analogen Verlauf mit umgekehrtem Vorzeichen der Geschwindigkeit.

Die Kurve 53 zeigt einen Beschleunigungsverlauf, wobei der Abschnitt 53a die Entleerungsphase und der Abschnitt 53b die Füllungsphase zeigt. In der Füllungsphase 53b ist der qualitative Verlauf aus Figur 2 zu erkennen. Die Füllungsphase 53b beginnt mit einer ersten Phase A weitgehend linear abnehmender Beschleunigung, weist dann eine zweite Phase B ohne Beschleunigung (a = 0) auf und endet mit einer dritten Phase C mit konstanter negativer Beschleunigung. Die Entleerungsphase 52a zeigt einen analogen Verlauf mit umgekehrtem Vorzeichen der Beschleunigung.

Der gezeigte Geschwindigkeits- und Beschleunigungsverlauf ermöglichen eine hohe Pumpleistung und einen schonenden Betrieb durch gleichmäßiges Pumpen der Membranblutpumpe 2 und eine ruckfreie Bewegung des Arbeitskolbens 6 sowie eine Verringerung der Gefahr der Blutschädigung.

Die Kurve 54 zeigt einen Relativdruckverlauf, wobei Abschnitt 54a die Entleerungsphase und Abschnitt 54b die Füllungsphase zeigt. Der Relativdruck nimmt am Anfang der Entleerungsphase 54a relativ schnell zu und bleibt dann etwa ab einem Drittel der Entleerungsphase 54a konstant hoch bis zum Ende der Entleerungsphase 54a. Ein umgekehrtes Verhalten ist in der Füllungsphase 54b zu sehen. Anhand des Relativdruckverlaufs wird deutlich, dass durch die spezielle Positionsvorgabe gemäß den Verläufen 51, 52 und 53 in einem überwiegenden Teil jeder Pumpzyklushälfte der Relativdruck weitgehend konstant ist. Ein konstant verlaufender Druck bietet den Vorteil, dass ausreichend Regelungsreserve zur Kompensation von Störungen vorhanden ist.

Die Kurve 55 zeigt einen Stromverlauf, wobei der Abschnitt 55a die Entleerungsphase und Abschnitt 55b die Füllungsphase zeigt. Auch der Strom bleibt durch die spezielle Positionsvorgabe gemäß den Verläufen 51, 52 und 53 über einen überwiegenden Teil einer Pumpzyklushälfte relativ konstant. Ein konstant verlaufender Strom ermöglicht ebenfalls ausreichend Regelungsreserve zur Kompensation von Störungen.

## Patentansprüche

1. Antriebsvorrichtung für eine Membranfluidpumpe, umfassend
eine Arbeitspumpe,
wobei die Arbeitspumpe zum Antreiben der Membranfluidpumpe über eine Druckleitung mit der Membranfluidpumpe verbunden ist, und wobei die Arbeitspumpe einen Hohlzylinder und einen darin axial zwischen zwei Umkehrpunkten oszillierbaren Arbeitskolben zum Verkleinern und Vergrößern eines mit der Druckleitung in einer Druckaustauschverbindung stehenden Arbeitsraums in der Arbeitspumpe aufweist, und
eine Steuereinheit zum Steuern einer Bewegung des Arbeitskolbens zwischen den Umkehrpunkten,
**dadurch gekennzeichnet, dass**
die gesteuerte Bewegung des Arbeitskolbens drei zeitlich aufeinanderfolgende Phasen umfasst, wobei
in einer ersten Phase der Arbeitskolben auf eine Geschwindigkeit beschleunigt wird, die größer ist als eine Geschwindigkeit am Ende der ersten Phase,
in einer zweiten Phase der Arbeitskolben derart bewegt wird, dass eine vorbestimmte Geschwindigkeit des Arbeitskolbens, ein vorbestimmter Relativdruck im Arbeitsraum oder eine vorbestimmte Kraft des Arbeitskolbens weitgehend konstant gehalten wird, und
in einer dritten Phase der Arbeitskolben mit einer negativen Beschleunigung bewegt wird.

2. Antriebsvorrichtung nach Anspruch 1, **dadurch gekennzeichnet, dass** die Steuereinheit eingerichtet ist, die Bewegung des Arbeitskolbens gemäß einer zeitlichen Positionsvorgabe für den Arbeitskolben zu steuern, wobei in der zweiten Phase die Geschwindigkeit des Arbeitskolbens weitgehend konstant gehalten wird, und/oder dass die Steuereinheit eingerichtet ist, die Bewegung des Arbeitskolbens gemäß einer zeitlichen Relativdruckvorgabe für den Arbeitsraum zu steuern, wobei in der zweiten Phase der Relativdruck im Arbeitsraum weitgehend konstant gehalten wird, und/oder dass die Steuereinheit eingerichtet ist, die Bewegung des Arbeitskolbens gemäß einer zeitlichen Kraftvorgabe für den Arbeitskolben zu steuern, wobei in der zweiten Phase die eine Kraft des Arbeitskolbens weitgehend konstant gehalten wird.

3. Antriebsvorrichtung nach einem der vorhergehenden Ansprüchen, **dadurch gekennzeichnet, dass** die Kraft des Arbeitskolbens eine Last und eine Trägheit des Arbeitskolbens einschließt.

4. Antriebsvorrichtung nach Anspruch 2, **dadurch gekennzeichnet, dass** die Steuereinheit eingerichtet ist, die zeitliche Positionsvorgabe basierend auf der zeitlichen Relativdruckvorgabe zu bestimmen, und die Bewegung des Arbeitskolbens gemäß der Positionsvorgabe zu steuern.

5. Antriebsvorrichtung nach einem der Ansprüche 2 bis 4, **dadurch gekennzeichnet, dass** die zeitliche Positionsvorgabe zusätzlich eine zeitliche Geschwindigkeitsvorgabe und/oder eine zeitliche Beschleunigungsvorgabe beinhaltet.

6. Antriebsvorrichtung nach einem der vorhergehenden Ansprüche, **dadurch gekennzeichnet, dass** die Arbeitspumpe ein Ausgleichsventil zum Ändern einer Luftmasse und/oder eines Relativdrucks im Arbeitsraum umfasst, und die Steuereinheit eingerichtet ist, das Ausgleichsventil gemäß einer Vorgabe für die Luftmasse und/oder einer Vorgabe für den Relativdruck zu steuern, insbesondere das Ausgleichsventil so zu steuern, dass die Luftmasse konstant gehalten wird und/oder das Ausgleichsventil gemäß einer Vorgabe für einen mittleren Relativdruck, gemäß einer Vorgabe für einen mittleren Entleerungsdruck oder gemäß einer Vorgabe für einen mittleren Füllungsdruck zu steuern, und/oder gemäß einer Relativdruckvorgabe und/oder einer Vorgabe für einen der beiden oder beide Umkehrpunkte zu steuern.

7. Antriebsvorrichtung nach einem der Ansprüche 2 bis 6, **dadurch gekennzeichnet, dass** die Steuereinheit eingerichtet ist, die zeitliche Positionsvorgabe basierend auf einer Referenztrajektorie für den Arbeitskolben zu bestimmen, wobei die Referenztrajektorie eine vorgegebene Dauer der ersten Phase, eine vorgegebene Dauer der zweiten Phase, eine vorgegebene Änderung der Beschleunigung des Kolbens in der ersten Phase, einen vorgegebenen Kolbenhub, einen vorgegebenen maximalen Relativdruck, eine vorgegebene Pumprate, eine vorgegebene relative Entleerungsdauer, einen vorgegebenen Füllungsgrad, einen vorgegebenen Entleerungsgrad, vorgegebene Kennwerte einer anzuschließenden Membranfluidpumpe, insbesondere ein Fördervolumen der Membranfluidpumpe, vorgegebene Kennwerte einer an die Membranfluidpumpe anschließbaren Einlasskanüle und/oder vorgegebene Kennwerte einer an die Membranfluidpumpe anschließbaren Auslasskanüle berücksichtigt.

8. Antriebsvorrichtung nach einem der Ansprüche 2 bis 7, **dadurch gekennzeichnet, dass** die Antriebsvorrichtung zum Antreiben des Arbeitskolbens einen elektrischen Spindelmotor aufweist, und dass die Steuereinheit eingerichtet ist, zum Steuern der Bewegung des Arbeitskolbens gemäß der zeitlichen Positionsvorgabe eine Antriebsstromstärke für den Spindelmotor basierend auf einem geschätzten erforderlichen Drehmoment für den Spindelmotor zu bestimmen, wobei in das Drehmoment eine Last des Arbeitskolbens, eine Trägheit des Arbeitskolbens, eine geschätzte Reibung des Arbeitskolbens und/oder eine Drehmomentkorrektur zur Kompensation einer Positionsabweichung und/oder einer Geschwindigkeitsabweichung des Arbeitskolbens von der zeitlichen Positionsvorgabe eingehen.

9. Antriebsvorrichtung nach dem vorhergehenden Anspruch, **dadurch gekennzeichnet, dass** mittels eines Positionssensors eine momentane Position und/oder eine momentane Geschwindigkeit des Arbeitskolbens während des Betriebs messbar sind und die Steuereinheit eingerichtet ist, eine Positionsabweichung und/oder eine Geschwindigkeitsabweichung von der zeitlichen Positionsvorgabe zu bestimmen, basierend auf der Positionsabweichung und/oder der Geschwindigkeitsabweichung die Drehmomentkorrektur zu bestimmen und die Antriebsstromstärke derart anzupassen, dass die Positionsabweichung und/oder die Geschwindigkeitsabweichung des Arbeitskolbens verringert werden.

10. Antriebsvorrichtung nach einem der vorhergehenden Ansprüche, **gekennzeichnet durch** eine Benutzerschnittstelle, mittels welcher benutzerspezifische Parameter, insbesondere ein Füllungs- und/oder Entleerungsgrad der Membranpumpe, ein Kolbenhub, ein mittlerer Relativdruck, eine relative Entleerungsdauer, ein Entleerungsdruck, ein Füllungsdruck und/oder eine Pumprate, während des Betriebs einstellbar sind.

11. Antriebsvorrichtung nach dem vorhergehenden Anspruch, **dadurch gekennzeichnet, dass** die Steuereinheit eingerichtet ist, die zeitliche Positionsvorgabe, die zeitliche Relativdruckvorgabe und/oder die zeitliche Kraftvorgabe für die Bewegungssteuerung des Arbeitskolbens und/oder die Vorgabe für die Luftmasse, die Vorgabe für den Relativdruck und/oder die Vorgabe für einen der beiden oder beide Umkehrpunkte für die Steuerung des Ausgleichsventils während des Betriebs an eine Änderung der benutzerspezifischen Parameter anzupassen.

12. Antriebsvorrichtung nach einem der Ansprüche 6 bis 11, **dadurch gekennzeichnet, dass** die Steuereinheit eingerichtet ist, einen Füllungsgrad der Membranpumpe durch Änderung eines Kolbenhubs und Steuern des Ausgleichsventils gemäß einer Vorgabe für den mittleren Entleerungsdruck im Arbeitsraum oder durch Änderung des mittleren Relativdrucks über das Ausgleichsventil einzustellen, und/oder einen Entleerungsgrad durch Änderung eines Kolbenhubs und Steuern des Ausgleichsventils gemäß einer Vorgabe für den mittleren Füllungsdruck im Arbeitsraum und/oder durch Änderung des mittleren Relativdrucks über das Ausgleichsventil einzustellen.

13. Pumpensystem, umfassend:
eine Membranfluidpumpe,
eine mit der Membranfluidpumpe verbundene Einlasskanüle zum Zuleiten eines Fluids zur Membranfluidpumpe sowie eine mit der Membranfluidpumpe verbundene Auslasskanüle zum Ableiten eines Fluids aus der Membranfluidpumpe,
**gekennzeichnet durch**
eine Antriebsvorrichtung für die Membranfluidpumpe nach einem der vorhergehenden Ansprüche.

14. Herzunterstützungssystem, umfassend:
eine Membranblutpumpe,
eine mit der Membranblutpumpe verbundene Einlasskanüle zum Zuleiten von Blut zur Membranblutpumpe aus einer Herzkammer und/oder einem Vorhof sowie eine mit der Membranblutpumpe verbundene Auslasskanüle zum Ableiten des Bluts aus der Membranblutpumpe in ein Blutgefäß,
**gekennzeichnet durch**
eine Antriebsvorrichtung für die Membranblutpumpe nach einem der Ansprüche 1 bis 12.

15. Verfahren zum Betrieb einer Antriebsvorrichtung für eine Membranfluidpumpe nach einem der Ansprüche 1 bis 12,
**dadurch gekennzeichnet, dass**
die Bewegung des Arbeitskolbens derart gesteuert wird, dass die Bewegung drei zeitlich aufeinanderfolgende Phasen umfasst, wobei in der ersten Phase der Arbeitskolben auf eine Geschwindigkeit beschleunigt wird, die größer ist als eine Geschwindigkeit am Ende der ersten Phase,
in der zweiten Phase der Arbeitskolben derart bewegt wird, dass eine vorbestimmte Geschwindigkeit des Arbeitskolbens, ein vorbestimmter Relativdruck im Arbeitsraum oder eine vorbestimmte Kraft des Arbeitskolbens weitgehend konstant gehalten wird, und
in der dritten Phase der Arbeitskolben mit einer negativen Beschleunigung bewegt wird.

16. Verfahren nach dem vorhergehenden Anspruch, **dadurch gekennzeichnet, dass** die Bewegung des Arbeitskolbens gemäß der zeitlichen Positionsvorgabe für den Arbeitskolben gesteuert wird, wobei in der zweiten Phase die Geschwindigkeit des Arbeitskolbens weitgehend konstant gehalten wird, und/oder dass die Bewegung des Arbeitskolbens gemäß der zeitlichen Relativdruckvorgabe für den Arbeitsraum gesteuert wird, wobei in der zweiten Phase der Relativdruck im Arbeitsraum weitgehend konstant gehalten wird, und/oder dass die Bewegung des Arbeitskolbens gemäß der zeitlichen Kraftvorgabe für den Arbeitskolben gesteuert wird, wobei die Kraft des Arbeitskolbens weitgehend konstant gehalten wird.

17. Verfahren nach dem vorhergehenden Anspruch, **dadurch gekennzeichnet, dass** die zeitliche Positionsvorgabe basierend auf der zeitlichen Relativdruckvorgabe bestimmt wird, und dass die Bewegung des Arbeitskolbens gemäß der zeitlichen Positionsvorgabe gesteuert wird.

18. Verfahren nach Anspruch 16, **dadurch gekennzeichnet, dass** die Bewegung des Arbeitskolbens in der ersten Phase und dritten Phase gemäß der zeitlichen Positionsvorgabe und in der zweiten Phase gemäß der zeitlichen Relativdruckvorgabe und/oder der zeitlichen Kraftvorgabe gesteuert wird.

19. Verfahren nach einem der drei vorhergehenden Ansprüche, **dadurch gekennzeichnet, dass** die Arbeitspumpe ein Ausgleichsventil zum Ändem einer Luftmasse und/oder eines Relativdrucks im Arbeitsraum umfasst,
wobei die Bewegung des Arbeitskolbens gemäß der zeitlichen Positionsvorgabe gesteuert wird und das Ausgleichsventil gemäß einer Vorgabe für den mittleren Relativdruck gesteuert wird, oder
wobei die Bewegung des Arbeitskolbens gemäß der zeitlichen Relativdruckvorgabe oder gemäß der zeitlichen Kraftvorgabe gesteuert wird und das Ausgleichsventil so gesteuert wird, dass die Luftmasse am Ende einer Entleerungsphase minimal ist.

20. Verfahren nach einem der Ansprüche 16 bis 19, **dadurch gekennzeichnet, dass** die zeitliche Positionsvorgabe basierend auf einer Referenztrajektorie für den Arbeitskolben bestimmt wird, wobei die Referenztrajektorie eine vorgegebene Dauer der ersten Phase, eine vorgegebene Dauer der zweiten Phase, eine vorgegebene Änderung der Beschleunigung des Arbeitskolbens in der ersten Phase, einen vorgegebenen Kolbenhub, einen vorgegebenen maximalen Relativdruck, eine vorgegebene Pumprate, eine vorgegebene relative Entleerungsdauer, einen vorgegebenen Füllungsgrad, einen vorgegebenen Entleerungsgrad, vorgegebene Kennwerte einer anzuschließenden Membranfluidpumpe, insbesondere ein Fördervolumen der Membranfluidpumpe, vorgegebene Kennwerte einer an die Membranfluidpumpe anschließbaren Einlasskanüle und/oder vorgegebene Kennwerte einer an die Membranfluidpumpe anschließbaren Auslasskanüle berücksichtigt.

21. Verfahren nach einem der vorhergehenden Ansprüche, **dadurch gekennzeichnet, dass** die Antriebsvorrichtung zum Antreiben des Arbeitskolbens einen elektrischen Spindelmotor aufweist, und dass zum Steuern der Bewegung des Arbeitskolbens gemäß der zeitlichen Positionsvorgabe eine Antriebsstromstärke für den Spindelmotor basierend auf einem geschätzten erforderlichen Drehmoment für den Spindelmotor bestimmt wird, wobei in das Drehmoment eine Last des Arbeitskolbens, eine Trägheit des Arbeitskolbens, eine geschätzte Reibung des Arbeitskolbens und/oder eine Drehmomentkorrektur zur Kompensation einer Positionsabweichung und/oder einer Geschwindigkeitsabweichung des Arbeitskolbens von der zeitlichen Positionsvorgabe eingehen.

22. Verfahren nach dem vorhergehenden Anspruch, **dadurch gekennzeichnet, dass** mittels eines Positionssensors eine momentane Position und/oder eine momentane Geschwindigkeit des Arbeitskolbens während eines Betriebs gemessen werden, und eine Positionsabweichung und/oder Geschwindigkeitsabweichung von der zeitlichen Positionsvorgabe bestimmt wird, basierend auf der Positionsabweichung und/oder der Geschwindigkeitsabweichung die Drehmomentkorrektur bestimmt wird und die Antriebsstromstärke derart angepasst wird, dass die Positionsabweichung und/oder die Geschwindigkeitsabweichung des Arbeitskolbens von der zeitlichen Positionsvorgabe verringert werden.

23. Verfahren nach einem der vorhergehenden Ansprüche, **dadurch gekennzeichnet, dass** benutzerspezifische Parameter, insbesondere ein Füllungs- und/oder Entleerungsgrad der Membranpumpe, ein Kolbenhub, ein mittlerer Relativdruck, eine relative Entleerungsdauer, ein Entleerungsdruck, ein Füllungsdruck und/oder eine Pumprate, während des Betriebs eingestellt werden und die zeitliche Positionsvorgabe, die zeitliche Relativdruckvorgabe und/oder die zeitliche Kraftvorgabe für die Bewegungssteuerung des Arbeitskolbens und/oder die Vorgabe für die Luftmasse, die Vorgabe für den Relativdruck und/oder die Vorgabe für die Luftmasse an einem der beiden oder an beiden Umkehrpunkten für die Steuerung des Ausgleichsventils während des Betriebs an eine Änderung der benutzerspezifischen Parameter angepasst werden.

24. Verfahren nach einem der Ansprüche 19 bis 23, **dadurch gekennzeichnet, dass** ein vorgegebener Füllungsgrad der Membranpumpe durch Änderung eines Kolbenhubs und Steuern des Ausgleichsventils gemäß einer Vorgabe für einen mittleren Entleerungsdruck im Arbeitsraum oder durch Änderung des mittleren Relativdruckes über das Ausgleichsventil eingestellt wird, und/oder ein vorgegebener Entleerungsgrad durch Änderung eines Kolbenhubs und Steuern des Ausgleichsventils gemäß einer Vorgabe für einen mittleren Füllungsdruck im Arbeitsraum und/oder durch Änderung des mittleren Relativdruckes über das Ausgleichsventil eingestellt wird.
